(19) Europäisches Patentamt
European Patent Office
Office européen des brevets

(11) **EP 2 288 907 B1**

(12) **EUROPEAN PATENT SPECIFICATION**

(45) Date of publication and mention
of the grant of the patent:
**05.09.2012 Bulletin 2012/36**

(51) Int Cl.:
**G01N 23/225** [(2006.01)]

(21) Application number: **09793444.2**

(22) Date of filing: **05.08.2009**

(86) International application number:
**PCT/ZA2009/000073**

(87) International publication number:
**WO 2010/022409 (25.02.2010 Gazette 2010/08)**

(54) **IDENTIFICATION OF PLATINUM GROUP MINERALS**

IDENTIFIKATION VON PLATINUMGRUPPENMINERALEN

IDENTIFICATION DE MINÉRAUX DU GROUPE PLATINE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **20.08.2008 ZA 200807173**

(43) Date of publication of application:
**02.03.2011 Bulletin 2011/09**

(73) Proprietor: **Mintek**
**2194 Randburg (ZA)**

(72) Inventor: **BUSHELL, Charles Laurence**
**2092 Johannesburg (ZA)**

(74) Representative: **Cabinet Plasseraud**
**52, rue de la Victoire**
**75440 Paris Cedex 09 (FR)**

(56) References cited:
**GB-A- 2 223 842     US-A- 4 476 386**

- H. M. PRICHARD, R.A.IXER, R.A. LORD, J. MAYNARD, N. WILLIAMS: "Assemblages of platinum-group minerals and sulfides in silicate lithologies and chromite-rich rocks within the shetland opholite" THE CANADIAN MINERALOGIST, vol. 32, 1994, pages 271-294, XP007910964 Retrieved from the Internet: URL: http://rruff.geo.arizona.edu/doclib/cm /vol32/CM32_271.pdf>
- XIAO Z ET AL: "Characterizing and recovering the platinum group minerals - A review" MINERALS ENGINEERING SEPTEMBER/OCTOBER 2004 ELSEVIER LTD GB, vol. 17, no. 9-10, September 2004 (2004-09), pages 961-979, XP002574389
- SEABROOK C L ET AL: "Platinum-group minerals in the Raglan Ni-Cu-(PGE) sulfide deposit, Cape Smith, Quebec, Canada" CANADIAN MINERALOGIST APRIL 2004 MINERALOGICAL ASSOCIATION OF CANADA CA, vol. 42, no. 2, April 2004 (2004-04), pages 485-497, XP002574390
- PETRUK W: "THE CAPABILITIES OH THE MICROPROBE KONTRON IMAGE ANALYSIS SYSTEM: APPLICATION TO MINERAL BENEFICIATION" SCANNING MICROSCOPY, SCANNING MICROSCOPY INTERNATIONAL, CHICAGO, IL, US, vol. 2, no. 3, 1 September 1988 (1988-09-01), pages 1247-1256, XP002040935 ISSN: 0891-7035

EP 2 288 907 B1

**Description**

BACKGROUND OF THE INVENTION

This invention relates to identifying platinum group minerals (PGMs) present in a sample.

Commercially available systems use scanning electron microscope (SEM) energy dispersive x-ray spectroscopy (EDS) to identify minerals present in a sample. (PRICHARD et al, The Canadian Mineralogist, vol. 32, 1994, 271-294).

At rest, atoms within the sample contain ground state (or unexcited) electrons in discrete energy levels or electron shells bound to the nucleus. An incident electron beam from the SEM will excite an electron in an inner shell, ejecting it from the shell while creating an electron hole where the electron was. An electron from an outer, higher-energy shell then fills the hole, and the difference in energy between the higher-energy shell and the lower energy shell is released in the form of an x-ray which is then detected and analyzed by the energy dispersive spectrometer. This x-ray is characteristic of the difference in energy between the two shills, and of the atomic structure of the element from which they were emitted. In this way, elements present in a sample can be identified, and from the elements present (and their proportions), a mineral identification can be made based on a known mineral chemical composition at each analysis point.

One system makes use of a "windowing" system wherein the EDS spectrum is divided into 32 windows, each 200-240 electron volts (eV) wide, and each assigned to an element. The total x-ray counts in the elemental windows are used to determine the presence of a particular element. A set of rules that a specific mineral must satisfy to be identified correctly (according to the total number of counts in the elemental windows) is established for all minerals in the sample. Using a "first match" approach, each analysis point is then compared to mineral identification rules until a match is found. If no match is found, the mineral is classified as unidentified.

In a second system the entire EDS spectrum from each sample is compared against a set of previously manually identified standard EDS spectra collected from minerals in the sample. Using a "best match" approach, each analysis point is matched to a mineral identification. If no match is found, the mineral is classified as unidentified.

Each system adequately identifies minerals in most mineral samples. However, in the case of PGM ores, misidentification of the PGMs often occurs. This is most often due to:

(1) the size of PGM grains. PGMs frequently occur in sizes of less than 5 micrometers (this value is given in terms of an equivalent circle diameter). When an EDS system analyses such a small grain, elements from the surrounding mineral are also detected, leading to a mixed EDS spectrum which is prone to non-identification;

(2) the chemical composition of PGMs is highly variable;

(3) the entire EDS spectrum is used for identification purposes. This makes it extremely difficult to define a set of standard rules or collect suitable standard spectra for mineral identification, given the effects of small grain size and of variable composition; and

(4) some partial elemental overlaps occur in EDS spectra (e.g. platinum and zirconium, lead and sulphur, calcium and tellurium). This presents a problem to the former system, in particular, as these overlaps often fall within the same 200-240 eV preset window, leading to uncertainty in elemental identification.

The automated acquisition of results is, however, compromised if each result has to be reviewed for correction. For example, in a PGM feed sample run, configured to find 100 PGM grains, acquisition and processing times are typically as follows:

Table 1

| Step | Average time in hours |
|---|---|
| Machine time taken to run 2 polished sections at a resolution of 1 μm | 8 hours |
| Time taken to process results for reporting | 1 hour |
| Time taken to manually review 50% of PGM grains and correct identification accuracies | 3:5 hours |
| Proportion of total job time lost to manual review | 28% |

It is an object of the present invention to provide a method of identifying platinum group minerals which, at least partly, addresses the aforementioned drawbacks and decreases the time required for verification.

SUMMARY OF THE INVENTION

The invention provides a method for identifying platinum group minerals (PGMs) which includes the steps of demarcating a plurality of spaced, successive, energy channels, each of a predetermined energy width, which span an energy spectrum of an energy dispersive x-ray spectrometer, establishing a reference table of normalised spectra of different PGM species, extracting from the reference table spectra count values, for each energy channel, only for elements found in, or associated with, PGMs, subjecting a sample mineral to a scanning electron microscope to produce an energy dispersive spectrum for the sample, and comparing the amplitude of the spectrum in a single channel against data extracted from the reference table to detect the presence of a particular element which is a known constituent of, or associated with, a PGM.

The width of each energy channel is 20 eV. As a standard EDS spectrum consists of 1024 energy channels the energy range is (1024) X (20 eV) = 20,480 keV.

BRIEF DESCRIPTION OF THE DRAWINGS

DESCRIPTION OF PREFERRED EMBODIMENT

In the method of the invention the identification of PGMs is conducted in an automated manner using a protocol which is conveniently referred to as narrow band selective elemental windowing.

A reference table of normalised spectra of different PGM species is established. EDS spectra are collected using standard SEM conditions to ensure that the spectra from different occurrences of the same mineral are always substantially the same. A total input spectrum x-ray count of 50,000 counts is selected and the total count is then normalised to 10,000 total counts using the formula:

$$\text{normalised count} = \frac{\text{channel x} - \text{ray count}}{\text{total spectrum x} - \text{ray count}} \times 10\,000 \,.$$

Normalisation ensures that elemental proportions of a particular PGM type remain relatively constant in terms of x-ray counts in an elemental channel from analysis to analysis irrespective of the number of total counts in the input spectrum.

Only certain elements that are known constituents of or associated with PGMs are considered and all other elements are ignored. This approach overcomes a major problem, associated with the second system referred to hereinbefore, namely the misidentification of minerals when mixed spectra are encountered. Mixed spectra are PGM spectra that include elements from host minerals. In such spectra misidentification is attributable, at least, to the small size of the PGM grains.

In the calibrated EDS system the apex of the peak always occurs at a particular energy for a particular element. For example, platinum occurs at 2,052 keV and zirconium at 2,042 keV.

The narrow band elemental window counts in the acquired EDS spectrum are then compared to a set of conditions for identification of the different PGM species, using a first match approach. The setup of these conditions is based on the prior analysis of numerous PGM spectra which are used to establish a reference table of normalised spectra. 10,000 total counts has been selected as the normalised value to avoid having to use decimal places for count ranges in the mineral identification module. When using a first match approach, the order of minerals in the set of identification conditions is carefully considered, and complex minerals are placed at the top of the list, with simpler ones lower down.

In the method of the invention a single 20 eV channel is located at the apex of the elemental x-ray peak which is the strongest channel for a particular element and only the counts within that channel are used to confirm the presence of the element.

Selective windowing means that only EDS energy peaks of elements found in PGMs, base metal sulphides and gangue respectively, which are present in PGM ores, are considered when identification is being performed. Other elements and, particularly, elements not found in PGMs, are ignored. As the entire spectrum is not looked at identification problems which arise when mixed spectra are analysed are avoided.

The channel which is chosen is at the centre of the elemental EDS peak which gives the highest count value. This helps to distinguish between common EDS overlaps of elements such as lead, which has a peak centre at 2,346 keV and sulphur, which has a peak centre at 2,308 keV. These peaks are only 38 eV apart and this can be partially resolved by a 20 eV window but not by a 140 eV window.

The method of the invention has been tested and shows better than 95% accuracy in PGM identification to a spatial resolution of 1 micron.

Table 2

| Step | Average time in hours |
|---|---|
| Machine time taken to run 2 polished sections at a resolution of 1 μm | 8 hours |
| Time taken to process results for reporting | 1 hour |
| Time taken to manually review 5% of PGM grains and correct identification accuracies | 20 min |
| Proportion of total job time lost to manual review | 3.7% |

Table 2 reflects the benefit of using the method of the invention and should be compared to Table 1. With the prior art approach the time taken for manual review of 50% of PGM grains is about three and a half hours for one system. In the method of the invention this is reduced to about twenty minutes where only about 5% of the PGM grains are reviewed.

The results produced by the method of the invention can be used in conjunction with other types of results produced by systems that measure a degree of liberation and grain sizes.

A comparison between the automated PGM identification system of the invention and documentation by means of manual identification was conducted, and a selection of the results thereof is presented in Table 3. The test was conducted as follows:

1. Four polished sections (labeled PRC 1, 2, 3 and 4) of a PGM flotation concentrate sample from a PGM producer were prepared. Three copper pins were also mounted in each section to act as reference points.

2. 165 PGM occurrences in total were located in the four polished sections. The location of each PGM in each section, and its position relative to the copper reference points, were recorded. A digital image of each PGM occurrence was acquired.

3. An EDS spectrum of each PGM was acquired and saved.

4. Each EDS spectrum obtained was then evaluated by using the method of the invention, providing an automated mineral identification for each PGM occurrence.

5. The four polished sections were then submitted for independent electron microprobe analyses. The location in the polished section and the digital image of each PGM were provided for easy re-location of the PGM grains.

6. An electron microprobe analysis of each PGM was performed, using wavelength dispersive spectroscopy (WDS) which provided a chemical composition at each analysis point.

7. The microprobe analyses were then manually evaluated by an experienced PGM mineralogist, who provided mineral names for each PGM grain, based on the chemical composition obtained.

8. The manual mineral identifications were then compared to the automated mineral identifications obtained in the preceding step 4.

The results showed that the method of the invention successfully identified 162 of the 165 PGM grains, representing a success rate of better than 98%.

The reasons for the three mis-identifications are as follows:

- In a first case a mixed spectrum of the PGM and its associated base metal sulphide (BMS) was acquired due to the small size of the PGM. The method of the invention did produce a result (PtFe, ferroplatinum) but this could not be manually confirmed by the microprobe result, due to the mixed spectrum.
- In a second case the method produced a PGM identification of PdTe (mineral equivalent: telluropalladinite) whilst the microprobe results returned a result of temagamite (PdHgTe). This represents a partial match. Temagamite is not a common PGM, and had not been added to the database at the time of the test, which is the reason for the mis-identification. Temagamite was subsequently added to the database, and the method now successfully identifies this PGM type.
- The third mis-identification was in respect of a grain with a chemical composition for which there is no known PGM match. Thus the method of the invention returned a result of "unknown", whilst the microprobe result could also not be manually associated with any known PGM mineral.

**Table 3: Comparison of Results - Method of Invention and Manual PGM Identification**

| Sample/PGM Number | Identiplat PGM Identification | Identiplat PGM Name | Manual PGM ID From Probe Results | Chemical Formula (Calculated From Probe Results) | Result |
|---|---|---|---|---|---|
| PRC1 PGM1 | PtPbCuNiFeS | Kharaelakite | Kharaelakite | $(Pt_{2.5}Fe_{1.7}Ni_{1.6}Cu_{1.6}Pb_{0.8})_{\Sigma=8.2}S_{8.8}$ | Match |
| PRC1 PGM2 | PtPdS | Braggite | Braggite | $Pt_{0.6}Pd_{0.3}Nt_{0.2}S_{0.9}$ | Match |
| PRC1 PGM3 | PtS | Cooperite | Cooperite | $PtFe_{0.1}S_{0.9}$ | Match |
| PRC1 PGM4 | PdS | Vysotskite | Vysotskite | $Pd_{0.6}Ni_{0.2}Fe_{0.2}S$ | Match |
| PRC1 PGM5 | PtRhCuS | Malanite | Rh-Malanite | $(Pt_{1.1}Rh_{0.5}Co_{0.2}Pd_{0.1})_{\Sigma=1.9}$ $(Cu_{0.9}Ni_{0.1}Fe_{0.1})_{\Sigma=1.1}S_4$ | Match |
| PRC1 PGM6 | PtS | Cooperite | Cooperite | $PtFe_{0.1}S_{0.9}$ | Match |
| PRC1 PGM7 | PtRhCuS | Malanite | Rh-Malanite | $(Pt_{1.0}Rh_{0.1}Co_{0.3}Ir_{0.1}Pd_{0.1})_{\Sigma=2.2}$ $(Cu_{0.9}Ni_{0.3}Fe_{0.2})_{\Sigma=1.4}S_{3.4}$ | Match |
| PRC2 PGM1a | PtS | Cooperite | Cooperite | $Pt_{0.8}Ni_{0.1}S_{1.1}$ | Match |
| PRC2 PGM1b | PtS | Cooperite | Cooperite | $Pt_{0.8}Rh_{0.1}Fe_{0.2}S_{0.9}$ | Match |
| PRC2 PGM2 | PtAs | Sperrylite | Sperrylite | $Pt_{0.9}Rh_{0.1}As_{1.8}S_{0.2}$ | Match |
| PRC3 PGM1 | PtPdFe | Ferroplatinum (Pd) | PtPdFe | $Pt_{0.5}Pd_{0.3}Fe_{0.2}$ | Match |
| PRC3 PGM3 | Ru(Os,Ir)S | Laurite | Laurite | $Ru_{0.7}Os_{0.2}Fe_{0.1}S_2$ | Match |
| PRC3 PGM6 | PtRhCuS | Malanite | Rh-Malanite | $(Pt_{1.0}Rh_{0.4}Co_{0.3}Ir_{0.2})_{\Sigma=1\cdot9}$ $(Cu_{0.9}Ni_{0.1}Fe_{0.1})_{\Sigma=1\cdot1}S_4$ | Match |
| PRC3 PGM7a | Ru(Os,Ir)S | Laurite | Laurite | $Ru_{0.7}Os_{0.1}Fe_{0.2}S_2$ | Match |
| PRC3 PGM7b | Ru(Os,Ir)S | Laurite | Laurite | $Ru_{0.7}Rh_{0.1}Fe_{0.2}S_2$ | Match |
| PRC4 PGM2 | PtS | Cooperite | Cooperite | $Pt_{0.4}Ni_{0.4}Fe_{0.2}S$ | Match |
| PRC4 PGM3 | PtPdS | Braggite | Braggite | $Pd_{0.7}Pt_{0.1}Ni_{0.2}S$ | Match |
| PRC4 PGM4 | PtS | Cooperite | Cooperite | $Pt_{0.9}(Ni,Fe)_{0.1}S$ | Match |
| PRC4 PGMS | Ru(Os,Ir)S | Laurite | Laurite | $Ru_{0.9}S_{2.1}$ | Match |
| PRC4 PGM6 | PtPdFe | Ferroplatinum (Pd) | Pd? | $Pd_{0.6}Pt_{0.3}Fe_{0.1}$ | Match |
| PRC4 PGM7 | PtS | Cooperite | Cooperite | $Pt_{0.6}Pd_{0.1}Ni_{0.1}Fe_{0.1}s_{1.1}$ | Match |
| PRC4 PGM8 | Unidentified | ??? | Rh | $Rh_{0.7}(Pt,Pd,Ir,Pb)_{0.3}$ | Unknown PGM |

**Claims**

1. A method for identifying platinum group minerals (PGMs) which includes the steps of demarcating a plurality of spaced, successive, energy channels, each of a predetermined energy width, which span an energy spectrum of an energy dispersive x- ray spectrometer, establishing a reference table of normalised spectra of different PGM species, extracting from the reference table spectra count values, for each energy channel, only for elements found in, or associated with, PGMs, subjecting a sample mineral to a scanning electron microscope to produce an energy dispersive spectrum for the sample, and comparing the amplitude of the spectrum in a single channel against data extracted from the reference table to detect the presence of a particular element which is a known constituent of, or associated with, a PGM, wherein the width of each energy channel is 20 eV.

2. A method according to claim 1 wherein, for a particular element, a single channel is located at the apex of the elemental x-ray peak which is the strongest channel for the element and only the counts within that channel are used to confirm the presence of the element.

**Patentansprüche**

1. Verfahren zum Identifizieren von Mineralien der Platingruppe (PGMs), das die Schritte einschließt: Abgrenzen mehrerer voneinander beabstandeter, aufeinanderfolgender Energiekanäle, von denen jeder eine vorgegebene Energiebreite hat, die ein Energiespektrum eines energiedispersiven Röntgenspektrometers abdecken; Erstellen einer Referenztabelle normalisierter Spektren verschiedener Arten von PGMs; Entnehmen von Spektrumzählerwerten aus der Referenztabelle für jeden Energiekanal nur für Elemente, die in PGMs gefunden werden oder mit ihnen verbunden sind; Behandeln einer Mineralprobe mit einem Rasterelektronenmikroskop, um ein energiedispersives Spektrum der Probe zu erzeugen, und Vergleichen der Amplitude des Spektrums in einem einzelnen Kanal mit Daten, die aus der Referenztabelle entnommen worden sind, um das Vorhandensein eines bestimmten Elements, von dem bekannt ist, das es ein Bestandteil eines PGM ist, oder mit diesem verbunden ist, zu erkennen, wobei die Breite jedes Energiekanals 20 eV beträgt.

2. Verfahren nach Anspruch 1 wobei für ein bestimmtes Element ein einzelner Kanal am Scheitelpunkt der elementaren Röntgenstrahlenspitze angeordnet ist, welches der stärkste Kanal für das Element ist, und wobei nur die Zählerereignisse innerhalb dieses Kanals benutzt werden, um das Vorhandensein des Elementes zu bestätigen.

**Revendications**

1. Procédé pour identifier des minéraux du groupe du platine (MGP) qui inclut les étapes de délimitation d'une pluralité de canaux d'énergie successifs espacés, chacun d'une largeur d'énergie prédéterminée, qui couvrent un spectre d'énergie d'un spectromètre à rayons X dispersif en énergie, établissement d'une table de référence de spectres normalisés de différentes espèces de MGP, extraction de la table de référence de valeurs de nombres de spectres, pour chaque canal d'énergie, seulement pour des éléments trouvés dans, ou associés avec, des MGP, exposition d'un échantillon minéral à un microscope électronique à balayage pour produire un spectre dispersif en énergie pour l'échantillon, et comparaison de l'amplitude du spectre dans un seul canal à des données extraites de la table de référence pour détecter la présence d'un élément particulier qui est un constituant connu de, ou associé avec, un MGP, où la largeur de chaque canal d'énergie est 20 eV.

2. Procédé selon la revendication 1 dans lequel, pour un élément particulier, un seul canal est situé au sommet du pic de rayons X élémentaire qui est le plus fort canal pour l'élément et seulement les nombres dans ce canal sont utilisés pour confirmer la présence de l'élément.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PRICHARD et al.** *The Canadian Mineralogist,* 1994, vol. 32, 271-294